# EUROPEAN PATENT APPLICATION

(11) **EP 4 814 803 A1**
(43) Date of publication of application: **30.09.2026**
(21) Application number: 26156841.4
(22) Date of filing: 06.02.2026
(51) Int. Cl.: G06N 3/08, G06N 3/044, G06N 10/60

(54) **A STRUCTURE FOR A MACHINE-LEARNING MODEL SUITABLE FOR RESERVOIR COMPUTING AND ITS APPLICATION TO PREDICT MATERIAL DEGRADATION**

(30) Priority: 24.03.2025 US 202519087938
(71) Applicant: Airbus (S.A.S.), 31700 Blagnac (FR); qBraid, Chicago, IL 60606 (US)
(72) Inventor: Tandon, Akshat, 31700 Blagnac (FR); Heitritter, Kenneth, Chicago, 60606 (US); Brown, James, Chicago, 60606 (US)
(74) Representative: KASTEL Patentanwälte PartG mbB

(57) **Abstract**

In order to improve that analysis and predictions of complex tasks such as material degradation prediction the invention proposes a machine learning model that is suitable for reservoir computing and comprises a computer-implemented input module configured for receiving input data; a plurality of reservoir networks, each reservoir network configured for receving data from the input module and for outputting reservoir state data; and computer-implemented readout module that is configured for receiving the reservoir state data from the reservoirs and for outputting readout data based on the reservoir state data. The reservoir networks can be computer-implemented or physically-implemented and at least one of them may involve a plurality of quibits.

## Description

The invention relates to a structure of a machine learning model that is suitable for reservoir computing, as well as methods for training and operating such a structure/machine learning model. The invention further relates to applications to predict material degradation.

Reference is made to the following documents:
[1] Domingo, L., Carlo, G. & Borondo, F. Taking advantage of noise in quantum reservoir computing. Sci Rep 13, 8790 (2023). https://doi.org/10.1038/s41598-023-35461-5
[2] Chen et al., Temporal Information Processing on Noisy Quantum Computers, https://doi.org/10.48550/arXiv.2001.09498.
[3] Tortorella, D., Micheli, A. (2024). Onion Echo State Networks. In: Wand, M., Malinovska, K., Schmidhuber, J., Tetko, I.V. (eds) Artificial Neural Networks and Machine Learning - ICANN 2024. ICANN 2024. Lecture Notes in Computer Science, vol 15025. Springer, Cham. https://doi.org/10.1007/978-3-031-72359-9_9.

[1] discloses quantum reservoirs for reservoir computing. The paper discloses results showing that the amplitude damping noise can be beneficial to machine learning, while the depolarizing and phase damping noises should be prioritized for correction. This critical result sheds new light into the physical mechanisms underlying quantum devices, providing solid practical prescriptions for a successful implementation of quantum information processing in nowadays hardware.
[2] discloses that the combination of machine learning and quantum computing has emerged as a promising approach for addressing previously untenable problems. Reservoir computing is an efficient learning paradigm that utilizes nonlinear dynamical systems for temporal information processing, i.e., processing of input sequences to produce output sequences. The paper discloses quantum reservoir computing that harnesses complex dissipative quantum dynamics. The class of quantum reservoirs is universal, in that any nonlinear fading memory map can be approximated arbitrarily closely and uniformly over all inputs by a quantum reservoir from this class. We describe a subclass of the universal class that is readily implementable using quantum gates native to current noisy gate-model quantum computers. Proof-of-principle experiments on remotely accessed cloud-based superconducting quantum computers demonstrate that small and noisy quantum reservoirs can tackle high-order nonlinear temporal tasks. The experimental results pave the path for attractive temporal processing applications of near-term gate-model quantum computers of increasing fidelity but without quantum error correction, signifying the potential of these devices for wider applications including neural modeling, speech recognition and natural language processing, going beyond static classification and regression tasks.
[3] discloses echo state networks (ESNs), which are a class of recurrent neural networks (RNNs) configured according to the Reservoir Computing (RC) approach, where the dynamical part of the model is initialized and left untrained. The topology of the reservoir and spectral properties both play an important role in producing an informative encoding of the input sequence. So far, only deep ESNs have been able to provide a clear hierarchy of representations by structuring the reservoir in multiple layers, thus encoding the input sequence on different time scales. In [3] an Onion ESNs is proposed, whose reservoirs are structured in segregated groups of units, each group corresponding to an annular segment of the whole reservoir spectrum. The experimental analysis of the model confirms that groups of reservoir units provide representations specialized in different dynamical regimes and signal frequencies. The results of this paper can be applied to the adaptive designs of ESNs as well as to the design of fully-trained RNNs.

Material degradation due to environmental exposure - such as corrosion, e.g., by tarnishing and pitting - is a pervasive challenge across industries. Environmental conditions, including temperature, humidity, and conductivity, are typical key drivers of corrosion processes. The onset and progression of corrosion are intricately linked to these macro-environmental factors, making it important to establish a clear correlation between them and material degradation. This understanding is particularly critical in high-stakes sectors like aerospace, where maintaining material integrity and reliability directly impacts safety, performance, and operational efficiency.

Various Al predictive models have been developed using supervised/unsupervized machine learning techniques in order to predict with the degree of corrosion as the target parameter. Such a dataset includes time-series data of environmental conditions and material degradation metrics, simulating real-world aerospace environments.

The Applicant has numerous initiatives that aim to predict and understand the evolution of corrosion by analyzing the impact of environmental conditions, aircraft age, and other contributing factors.

Leveraging Al solutions in these projects is pivotal for driving innovation in material longevity and predictive maintenance. Such advancements align with sustainability goals by reducing material waste, optimizing manufacturing processes, and minimizing maintenance costs. These efforts not only enhance operational efficiency but also contribute to the long-term reliability and environmental responsibility of aircraft of the future.

It is the object of the invention to provide or improve systems and methods that allow predictions of complex processes, such as material degradation.

The object is achieved by the subject-matter of the independent claims. Preferred embodiments are subject-matter of the dependent claims.

The invention provides a structure of a machine learning model, the structure being suitable for reservoir computing and comprising:
- a computer-implemented input module configured for receiving input data;
- a plurality of reservoir networks, each reservoir network configured for receving data from the input module and for outputting reservoir state data;
- a computer-implemented readout module that is configured for receiving the reservoir state data from the reservoirs and for outputting readout data based on the reservoir state data.

This architecture of a machine learning model specifically allows for use of the model in a reservoir computing approach, which enables efficient processing of complex, time-dependent data. The architecture involves of three key components: an input module, a plurality of reservoir networks, and a readout module.

The input module is a computer-implemented module preferably configured for receiving raw input data from an external source, such as sensor readings, time-series measurements, or other real-world signals. It preprocesses and transforms the incoming data into a format compatible with the subsequent reservoir networks. Since reservoir networks may differ in structure and computational properties, the input module ensures that the data is appropriately formatted, normalized, or encoded to maintain consistency and facilitate effective processing across multiple reservoirs.

The system comprises multiple reservoir networks, each receiving processed data from the input module. These reservoirs act as dynamic memory systems, capturing temporal dependencies and preserving information over time, much like short-term and long-term memory. The reservoirs are preferably heterogeneous, meaning they can differ in type, structure, and operational principles. Some reservoirs may be classical, such as echo state networks or recurrent neural networks, while others may be quantum-based, utilizing qubit-based reservoirs. Within the quantum category, reservoirs may operate using different modalities, such as superconducting qubits, trapped ions, or neutral atom qubits. Each reservoir is preferably configured for a specific (memory-)function, with at least one of the reservoirs emphasizing long-term memory by sustaining dynamics over longer timescales, while other reservoirs focus on short-term memory by capturing transient, immediate changes. These reservoirs collectively create a hybrid computational environment, allowing to leverage the advantages of both classical and quantum memory mechanisms.

The readout module is a computer-implemented component responsible for interpreting and extracting meaningful output from the collective reservoir states. Since reservoir networks encode information in high-dimensional, often abstract state representations, the readout module translates these states into a human-understandable form. The final output may correspond to measurable physical parameters, such as corrosion or material degradation predictions based on environmental factors, anomaly detection in time-series data, or pattern recognition tasks where the system detects trends and categorizes events based on reservoir-processed signals. The readout module may employ machine learning models, such as linear regression, neural networks, or decision trees, to map reservoir states to final predictions. Depending on the application, the module can be designed for real-time decision-making, enabling predictive maintenance, anomaly detection, or other automated responses.

This structured approach offers multiple key advantages. It should be noted that not all advantages may be present at the same time or with similar intensity. By integrating diverse reservoirs, the architecture supports both short-term and long-term memory retention, mimicking the multi-scale memory behavior of the human brain. The inclusion of a dedicated input processing unit ensures that heterogeneous reservoirs receive data in a standardized manner, optimizing efficiency and stability. The system is flexible and scalable, allowing for the integration of both classical and quantum computing elements, making it adaptable to a wide range of applications. Finally, the readout module ensures that the complex internal states of the reservoir networks are transformed into actionable insights, bridging the gap between raw computational processes and human-readable outputs.

Preferably, each reservoir network is separated from the other reservoir networks.

Preferably, each reservoir network is configured to receive the same input data as the other reservoir networks.

Preferably, each reservoir network is configured as an echo state network.

Preferably, each reservoir network is sparsely connected. Preferably each reservoir network is connected with a sparsity factor of 5% to 20%. In other words preferably a fraction of 5% to 20% of all possible connections have a nonzero weight. Preferably, a fraction of 80% to 95% of all possible connections have zero weight.

Preferably, the plurality of reservoir networks includes a computer-implemented first reservoir network and a computer-implemented second reservoir network.

Preferably, the plurality of reservoir networks includes a computer-implemented first reservoir and a physically-implemented second reservoir that is configured as a quantum reservoir and formed by a plurality of qubits.

Preferably, the plurality of reservoir networks includes a physically-implemented first reservoir and a physically-implemented second reservoir, each reservoir being configured as a quantum reservoir, wherein the first reservoir is formed by a first type of qubits and the second reservoir is formed by a second type of qubits that is different from the first type of qubits.

Preferably, the plurality of reservoir networks includes a computer-implemented first reservoir network and a computer-implemented second reservoir network, wherein at least one of the first and second reservoir networks is implemented as a quantum reservoir by means of program code that emulates a plurality of qubits.

Preferably, at least one of the reservoirs is configured as a short-term memory reservoir. Preferably, the weight matrix of that reservoir is configured such that the absolute values of the eigenvalues of that weight matrix are in the range of 0.0 to 0.4.

Preferably, at least one of the reservoirs is configured as a long-term memory reservoir. Preferably, the weight matrix of that reservoir is configured such that the absolute values of the eigenvalues of that weight matrix are in the range of 0.8 to 1.0.

In general it is allowed that reservoirs that are configured as shorter term memory can have a higher noise in the qubits or other parts of the system. This corresponds to a lower qubit fidelity or overall fidelity. In contrast it is preferred that reservoirs that are configured as longer term memory involve qubits that have lower noise, and thus higher qubit fidelity or overall fidelity.

In other embodiments it is possible to combine qubits of different fidelity, mainly for the purpose of maximizing the amount of available qubits as the application may require. It should be noted that in this case the quantum fidelity of the respective reservoir is typically lower, but this disadvantage - if it affects the method at all - is more than made up for by the significantly larger availability of qubits for said reservoir.

Preferably, the weight matrix of each reservoir is configured such that the absolute values of the eigenvalues of that weight matrix are not in the range of 0.5 to 0.7.

Preferably, the readout module includes a concatenation layer that is configured to concatenate the reservoirs state data of all reservoirs networks. Preferably, the readout module includes a readout network layer that is configured to receive the concatenated reservoir state data and generate the readout data.

The invention provides a computer-implemented method for supervised training of a previously described structure to obtain a trained machine-learning model, the method comprising performing a supervised training method on the structure, wherein the parameters of each reservoir network are randomly initialized and kept static during training, wherein the parameters of the readout module are updated during training.

Preferably, architecture and/or hyperparameters of each reservoir network are randomly initialized.

The invention provides a machine learning model obtainable by a previously described supervised training method.

The invention provides a computer-implemented method for operating a previously described machine learning model, wherein sequential data is fed as input data into the machine learning model which determines the readout data as output data.

The invention provides a computer-implemented method for supervised training of a previously described structure to obtain a material degradation predictor model, the method comprising performing a previously described training method, wherein labeled training data are used in training the material degradation predictor model, wherein the labeled training data include environmental data and degradation data, wherein the environmental data include at least one time sequence of a physical parameter of an environment to which a material sample is exposed, the physical parameter influencing material degradation, wherein the degradation data are a label indicate a state of degradation of the material sample.

The invention provides a material degradation predictor model obtainable by above method.

The invention provides a computer-implemented method for determining material degradation of a material sample with a material degradation predictor model, the method comprising feeding sequential environmental data into the material degradation predictor model which estimates a state of degradation of the material sample based on the environmental data.

The invention provides labeled training data that are suitable for training a previously described structure as a material degradation predictor model, wherein the training data include a plurality of training samples, and each training sample has environmental data and degradation data, wherein the environmental data include at least one time sequence of a physical parameter of an environment to which a material sample is exposed, the physical parameter influencing material degradation, wherein the degradation data are a label and indicate a state of degradation of the material sample.

The invention provides a data processing apparatus comprising means to perform any of the preceding computer-implemented methods.

The invention provides a computer program that includes instructions that, when executed by a data processing apparatus, cause the data processing apparatus to perform any of the preceding computer-implemented methods.

The invention provides a computer-readable data storage or data carrier signal comprising the computer program.

The proposed solution introduces hybrid quantum-classical and hybrid quantum-onion reservoir architectures that are configured to address computational challenges requiring both short-term and long-term memory, as well as high computational fidelity. These architectures integrate multiple reservoir types, including classical and quantum reservoirs, structured to optimize computational efficiency and dynamic memory retention.

The hybrid onion reservoir is preferably a layered structure that integrates at least two distinct reservoir types to emulate short-term and long-term memory, enabling complementary effects essential for time-dependent applications such as signal processing, predictive modeling (e.g. for material degradation), and temporal pattern recognition. One layer, referred to as the higher complex eigenvalues reservoir, is configured with complex eigenvalues (i.e. values that have a real and imaginary part) having higher absolute values, which are chosen to sustain state dynamics over longer time scales and enhance long-term memory retention. This is particularly suitable for applications that require temporal persistence, such as system monitoring. The other layer, may be referred to as the lower complex eigenvalues reservoir, and is configured with eigenvalues having lower absolute values, which are chosen for capturing immediate and transient dynamics, thereby facilitating short-term memory. This component is particularly effective in tasks involving short-term prediction. By combining these two layers, the hybrid onion reservoir architecture enables a balanced memory profile, advantageously maintaining both adaptability to new data and robustness in long-range dependencies.

In addition to the hybrid onion reservoir, a hybrid classical-quantum reservoir architecture may integrate both classical neural networks and quantum reservoirs, leveraging the unique computational advantages of quantum processing while maintaining stability through classical components. This architecture is illustrated with two primary configurations. In the first configuration, a classical reservoir with higher complex eigenvalues is used for long-term memory, while a quantum reservoir with lower complex eigenvalues is used for short-term memory. In the second configuration, the classical reservoir has lower complex eigenvalues for short-term memory, while the quantum reservoir has higher complex eigenvalues for long-term memory. This hybrid approach capitalizes on quantum advantages such as parallelism and entanglement, while retaining the stability and scalability of classical networks. The interaction between classical and quantum reservoirs ensures dynamic information processing, making the system robust for sequential learning tasks.

Extending the principles of the hybrid onion reservoir into the quantum domain, the hybrid quantum-onion reservoir allows to combine different types of quantum processing units to simulate both short-term and long-term memory. This design enables adaptive tuning of quantum system dynamics by leveraging distinct quantum hardware properties. The analog quantum hybrid reservoir integrates analog quantum computing platforms, particularly superconducting and neutral atom qubits, each offering different fidelities and dynamic behavior. One configuration employs superconducting qubits from D-Wave (https://www.dwavequantum.com/), which offer higher fidelity and are designed for long-term memory, while neutral atom qubits from QuEra (https://www.quera.com/) provide lower fidelity and are optimized for short-term memory. An alternative configuration reverses these roles, using superconducting qubits for short-term memory and neutral atom qubits for long-term memory. These configurations allow controlled memory persistence and adaptability, leveraging the fast coherence of neutral atom qubits and the stable, high-fidelity dynamics of superconducting qubits.

Another variation of the hybrid quantum-onion reservoir, referred to as the gate-based quantum hybrid reservoir, combines gate-based quantum computing platforms, each offering distinct computational fidelity levels. One configuration employs superconducting qubits from IBM, which provide higher fidelity for long-term memory, alongside trapped ion qubits from lonQ, which offer lower fidelity for short-term memory. The alternative configuration reverses these roles, using superconducting qubits for short-term memory and trapped ion qubits for long-term memory. By integrating superconducting qubits with trapped ion qubits, this approach balances computational precision with diverse quantum interactions, offering greater flexibility in reservoir tuning.

A further enhancement in gate-based quantum reservoir computing is the use of mid-circuit qubit measurements to fine-tune the eigenvalues of the system dynamically. This methodology enables precise control over system eigenvalues based on the number of qubits measured in a circuit, utilization of multiple quantum processing units with different circuits that measure varying numbers of qubits mid-execution, and finer granularity in eigenvalue distribution. This allows for an adjustable memory profile suited to specific computational tasks. This approach extends the potential of hybrid quantum-classical systems, offering dynamic eigenvalue adaptation that can be optimized for different machine learning and computational applications.

The proposed hybrid quantum-classical and hybrid quantum-onion reservoir architectures provide a scalable, high-fidelity solution for time-dependent learning problems by integrating layered memory effects through onion reservoir structures, quantum-classical synergy to leverage the strengths of both paradigms, and dynamic quantum tuning via mid-circuit measurement techniques. By bridging the gap between classical computing limitations and quantum computational advantages, these architectures set a foundation for next-generation reservoir computing models with unparalleled efficiency and adaptability.

Furthermore, the whole system is typically configured as a distributed computer system, as at the time of filing suitable physically-implemented qubits are typically available by only a few providers. However, it should be noted that the system may operate in a distributed or non-distributed manner.

Although the invention is illustrated with its application towards material degradation prediction, it should be noted that other applications of the ideas disclosed herein are possible.

Embodiments of the invention are described in more detail with reference to the accompanying schematic drawings that are listed below
Fig. 1 depicts a first embodiment of a structure for a material degradation prediction model;
Fig. 2 depicts a second embodiment of a structure for a material degradation prediction model;
Fig. 3 depicts a third embodiment of a structure for a material degradation prediction model;
Fig. 4 depicts the trained material degradation prediction model at inference time.

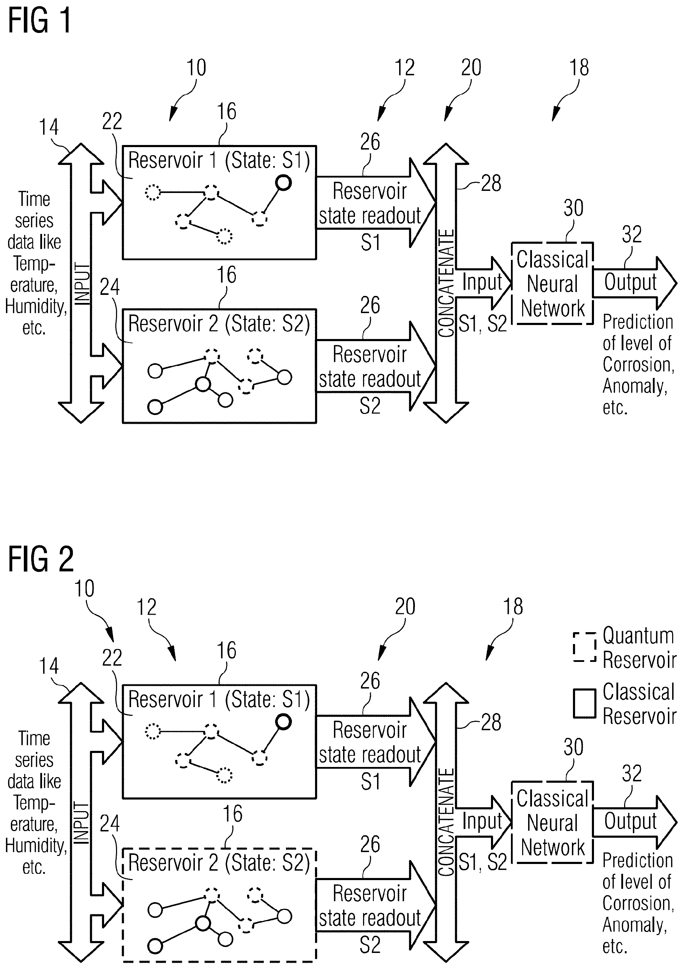

Referring to Fig. 1 a structure 10 for a machine learning model 12 is depicted. The structure 10 is sometimes also referred to as architecture. The structure 10 includes an input module 14, a plurality of reservoir networks 16, and a readout module 18. The machine learning model 12 is preferably a material degradation prediction model 20 that is configured for determining a degree of material degradation in a material sample.

The input module 14 is configured to receive time-series data as input data. With respect to determining a degree of material degradation, the input data may include temperature data (related to the environment and/or the material sample), humidity data, or any other physical parameter of the environment that is known to affect material degradation, specifically corrosion.

The reservoir networks 16 include a computer-implemented first reservoir network 22 and a computer-implemented second reservoir network 24. The first and reservoir networks 22, 24 differ in at least one characteristic property. The first reservoir network 22 is characterized by a weight matrix that has complex eigenvalues that have high absolute values, e.g. from 0.8 to 1.0. The second reservoir network 24 is characterized by a weight matrix that has complex eigenvalues that have low absolute values, e.g. from 0.0 to 0.4. Both the first and second reservoir networks 22, 24 are configured as sparsely connected networks, e.g. with only 5% to 20% of all possible connections having a nonzero weight. Each reservoir network 16 has as a final layer a non-linear function layer, such as sigmoid, tanh, or ReLU, as is known in the field. Each reservoir network 16 outputs reservoir state data 26, which include the activations of all neurons within the respective reservoir network 16.

The readout module 18 is computer-implemented and includes a concatenation layer 28 that is configured to concatenate the reservoir state data 26 of all reservoir networks 16.

The readout module 18 further comprises a readout network layer 30 that may be configured as a simple or deep neural network. Preferably the readout network layer 30 is configured as a fully connected deep neural network. The readout network layer 30 includes an output layer 32.

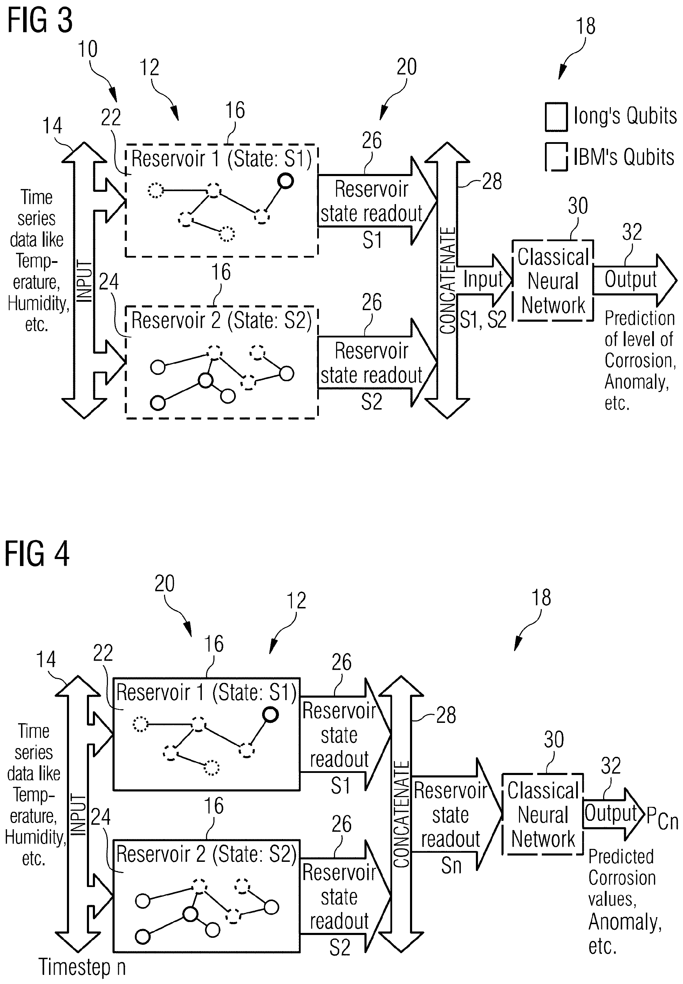

Referring to Fig. 2, the structure 10 is basically the same as the of the first embodiment. In contrast to the first embodiment, the second reservoir network 24 is not computer-implemented but rather physically-implemented as a quantum reservoir made form qubits. For details, reference is made to [1] which explains an implementation of a suitable quantum reservoir in detail that is not repeated herein for sake of brevity. The reservoir network 24 may be chosen from a sparsely connected, densely connected or fully connected network type. It is preferred to have at least a densely connected reservoir network 24. Referring to Fig. 3, the structure 10 is different from the first and second embodiments in that both the first and second reservoir networks 22, 24 are physically-implemented as a quantum reservoir.

In the following the training of the structure 10 is described in more detail. A set of labeled training data has to be generated. The training data are typically generated experimentally, by exposing a material sample to different environmental conditions and periodically measuring the degree of degradation, such as corrosion. The material sample may be scratched or the surface otherwise damaged prior to exposing. The degree of degradation can be measured by a usual method of the filed. In a very simple way, the degree of degradation can be measured via images and counting the number of pixels that show discoloration. The training data include time-series data of environmental data (such as temperature, humidity, etc.) which affect material degradation, specifically corrosion, of the material sample. Furthermore, the labels in the training data include a degree of corrosion. The degree of corrosion can be measured by several parameters that relate to the corrosion, such as affected area, loss mass, size or size distribution of affected areas, amount of pitting, etc. It is possible to automate at least some of the measurements.

At the beginning of training, the reservoir networks 16 are randomly initialized. The reservoir networks 16 may have hyperparameters that are randomly chosen, such as the number of layers, the number of neurons in a specific layer and the sparsity factor for the connections. Furthermore, the weights, bias, and/or initial neuron activation can be chosen randomly. It should be noted that the term randomly does not necessarily mean in this context that the values are chosen from an arbitrary range, rather some conditions or constraints are imposed, such as the eigenvalue constraints. The readout network layer 30 may also be randomly initialized.

After initialization, the parameters of the reservoir networks 16 are kept static, while the parameters of the readout network layer 30 are updated according to the usual backpropagation algorithm.

The first timestep of the training data is fed forward through the reservoir networks 16 and the readout module 18. The loss between the obtained prediction and the label in the training data is calculated, e.g., a cross-entropy loss or root-mean-square loss. Subsequently, the readout module 18 is updated by backpropagation based on the loss.

The next timestep of the training data is fed forward through the reservoir networks 16 and the readout module 18, wherein the reservoir state data produced by the previous timestep is updated. The updated reservoir state data are fed through the readout module 18 and the backpropagation is applied to the readout module 18. This process is repeated until an acceptable loss value or other abortion criterion (which may include running out of training data) is reached. Thus, the structure 10 is trained to be a material degradation prediction model 20.

Referring to Fig. 4, the operation material degradation prediction model 20 is described in more detail. The material degradation prediction model 34 may be installed on an aircraft (not shown) or other vehicle, for example. The material degradation model 34 receives timeseries data from sensors that are installed on the aircraft, e.g., temperature and humidity sensors. Based on the incoming sensor data, the material degradation prediction model 34 predicts a degree of material degradation. This value may be logged for later use during routine maintenance, or, if it exceeds a predetermined threshold, cause a message to the flight crew that a certain portion of the aircraft is in need of maintenance or should be inspected.

### List of reference signs:

- 10: structure
- 12: machine learning model
- 14: input module
- 16: reservoir network
- 18: readout module
- 20: material degradation prediction model
- 22: first reservoir network
- 24: second reservoir network
- 26: reservoir state data
- 28: concatenation layer
- 30: readout network layer
- 32: output layer

## Claims

1. A structure of a machine learning model, the structure being suitable for reservoir computing and comprising:
- a computer-implemented input module configured for receiving input data;
- a plurality of reservoir networks, each reservoir network configured for receving data from the input module and for outputting reservoir state data;
- a computer-implemented readout module that is configured for receiving the reservoir state data from the reservoirs and for outputting readout data based on the reservoir state data.

2. The structure of claim 1, wherein each reservoir network is separated from the other reservoir networks; and/or wherein each reservoir network is configured to receive the same input data as the other reservoir networks; and/or wherein each reservoir network is configured as an echo state network; and/or wherein each reservoir network is sparsely connected, preferably with a fraction of 5% to 20% of all possible connections having a nonzero weight and/or with a fraction of 80% to 95% of all possible connections having zero weight.

3. The structure of any of the preceding claims, wherein the plurality of reservoir networks includes any of the following:
a) a computer-implemented first reservoir network and a computer-implemented second reservoir network; and/or
b) a computer-implemented first reservoir and a physically-implemented second reservoir that is configured as a quantum reservoir and formed by a plurality of qubits; and/or
c) a physically-implemented first reservoir and a physically-implemented second reservoir, each reservoir being configured as a quantum reservoir, wherein the first reservoir is formed by a first type of qubits and the second reservoir is formed by a second type of qubits that is different from the first type of qubits; and/or
d) the plurality of reservoir networks includes a computer-implemented first reservoir network and a computer-implemented second reservoir network, wherein at least one of the first and second reservoir networks is implemented as a quantum reservoir by means of program code that emulates a plurality of qubits.

4. The structure of any of the preceding claims, wherein at least one of the reservoirs is configured as a short-term memory reservoir, wherein preferably the weight matrix of that reservoir is configured such that the absolute values of the eigenvalues of that weight matrix are in the range of 0.0 to 0.4; and/or wherein at least one of the reservoirs is configured as a long-term memory reservoir, wherein preferably the weight matrix of that reservoir is configured such that the absolute values of the eigenvalues of that weight matrix are in the range of 0.8 to 1.0; and/or wherein the weight matrix of each reservoir is configured such that the absolute values of the eigenvalues of that weight matrix are not in the range of 0.5 to 0.7.

5. The structure of any of the preceding claims, wherein the readout module includes a concatenation layer that is configured to concatenate the reservoirs state data of all reservoir networks, wherein the readout module includes a readout network layer that is configured to receive the concatenated reservoir state data and generate the readout data.

6. A computer-implemented method for supervised training of a structure of any of the preceding claims to obtain a trained machine-learning model, the method comprising performing a supervised training method on the structure, wherein the parameters of each reservoir network are randomly initialized and kept static during training, wherein the parameters of the readout module are updated during training.

7. A machine learning model obtainable by a supervised training method of claim 6.

8. A computer-implemented method for operating a machine learning model of claim 7, wherein sequential data is fed as input data into the machine learning model which determines the readout data as output data.

9. A computer-implemented method for supervised training of a structure of any of the claims 1 to 5 to obtain a material degradation predictor model, the method comprising performing the method of claim 6, wherein labeled training data are used in training the material degradation predictor model, wherein the labeled training data include environmental data and degradation data, wherein the environmental data include at least one time sequence of a physical parameter of an environment to which a material sample is exposed, the physical parameter influencing material degradation, wherein the degradation data are a label indicate a state of degradation of the material sample.

10. A material degradation predictor model obtainable by a method of claim 9.

11. A computer-implemented method for determining material degradation of a material sample with a material degradation predictor model of claim 10, the method comprising feeding sequential environmental data into the material degradation predictor model which estimates a state of degradation of the material sample based on the environmental data.

12. A data processing apparatus comprising means to perform any of the preceding computer-implemented methods of claim 6, 8, 9 or 11.

13. A computer program that includes instructions that, when executed by a data processing apparatus, cause the data processing apparatus to perform any of the preceding computer-implemented methods of claim 6, 8, 9 or 11.

14. A computer-readable data storage or data carrier signal comprising the computer program of claim 13.
